(19) Europäisches Patentamt
European Patent Office
Office européen des brevets

(11) **EP 1 372 652 B1**

(12) **EUROPEAN PATENT SPECIFICATION**

(45) Date of publication and mention
of the grant of the patent:
**21.05.2008 Bulletin 2008/21**

(51) Int Cl.:
*A61K 31/453* (2006.01)     *A61P 35/00* (2006.01)

(21) Application number: **02737950.2**

(22) Date of filing: **22.03.2002**

(86) International application number:
**PCT/EP2002/004083**

(87) International publication number:
**WO 2002/076484 (03.10.2002 Gazette 2002/40)**

(54) **COMBINATION OF DOCETAXEL AND FLAVOPIRIDOL**

KOMBINATION VON DOCETAXEL MIT FLAVOPIRIDOL

ASSOCIATION DE DOCETAXEL ET DE FLAVOPIRIDOL

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.03.2001 US 277948 P**
**05.07.2001 US 302692 P**
**04.12.2001 US 334916 P**

(43) Date of publication of application:
**02.01.2004 Bulletin 2004/01**

(73) Proprietor: **Aventis Pharma S.A.**
**92160 Antony (FR)**

(72) Inventor: **BISSERY, Marie-Christine**
**F-94400 Vitry sur Seine (FR)**

(74) Representative: **Le Pennec, Magali**
**Aventis Pharma S.A.**
**Direction des Brevets,**
**Tri LEO/144**
**20, avenue Raymond Aron**
**92165 Antony Cedex (FR)**

(56) References cited:
**WO-A-97/30174**

- SCHWARTZ G K ET AL: "PHASE I TRIAL OF SEQUENTIAL PACLITAXEL AND CISPLATIN IN COMBINATION WITH THE CYCLIN DEPENDENT KINASE INHIBITOR FLAVOPIRIDOL (FLAVO) IN PATIENTS WITH ADVANCED SOLID TUMORS" CLINICAL CANCER RESEARCH, THE AMERICAN ASSOCIATION FOR CANCER RESEARCH, US, vol. 5, no. SUPPL, November 1999 (1999-11), page 3754, AN122 XP001108950 ISSN: 1078-0432
- MOTWANI M ET AL: "SEQUENTIAL DEPENDENT ENHANCEMENT OF CASPASE ACTIVATION AND APOPTOSIS BY FLAVOPIRIDOL ON PACLITAXEL-TREATED HUMAN GASTRIC AND BREAST CANCER CELLS" CLINICAL CANCER RESEARCH, THE AMERICAN ASSOCIATION FOR CANCER RESEARCH, US, vol. 5, no. 7, July 1996 (1996-07), pages 1876-1883, XP001113185 ISSN: 1078-0432
- BIBLE K C ET AL: "CYTOXIC SYNERGY BETWEEN FLAVOPIRIDOL (NSC 649890, I86-8275) AND VARIOUS ANTINEOPLASTIC AGENTS: THE IMPORTANCE OF SEQUENCE OF ADMINISTRATION" CANCER RESEARCH, AMERICAN ASSOCIATION FOR CANCER RESEARCH, BALTIMORE, MD, US, vol. 57, 15 August 1997 (1997-08-15), pages 3375-3380, XP002128085 ISSN: 0008-5472
- MOTWANI M V ET AL: "DOCETAXEL AND NAVELBINE INDUCED APOPTOSIS IS ENHANCED BY FLAVOPIRIDOL (FLAVO) IN BREAST CANCER CELLS AND IS SEQUENCE DEPENDENT" PROCEEDINGS OF THE ANNUAL MEETING OF THE AMERICAN ASSOCIATION FOR CANCER RESEARCH, NEW YORK, NY, US, vol. 41, March 2000 (2000-03), page 143, AN912 XP008008541 ISSN: 0197-016X

• KELLAND L R: "FLAVOPIRIDOL, THE FIRST CYCLIN-DEPENDENT KINASE INHIBITOR TO ENTER THE CLINIC: CURRENT STATUS" EXPERT OPINION ON INVESTIGATIONAL DRUGS, ASHLEY PUBLICATIONS LTD., LONDON, GB, vol. 9, no. 12, December 2000 (2000-12), pages 2903-2911, XP008008516 ISSN: 1354-3784

Remarks:
The file contains technical information submitted after the application was filed and not included in this specification

**Description**

The present invention relates to combinations of Taxotere® and Flavopiridol which are therapeutically useful in the treatment of neoplastic diseases.

Taxanes and taxoids constitute a family of naturally occurring diterpene compounds including a potent antitumor drug, paclitaxel. Paclitaxel (Taxol®), originally isolated from the bark of the Pacific Yew tree (*Taxus brevifolia*), has been shown to be highly effective in adjuvant and neo-adjuvant therapies for patients with breast and ovarian cancers. More recently, its semisynthetic analogue, docetaxel (Taxotere®), has also been found effective in breast cancer chemotherapy, which has expanded the number of diseases sensitive to this class of antitumor drugs, including lung and colon cancers. Both Taxol® (paclitaxel) and Taxotere® (docetaxel) bind to tubulin, inhibit microtubule disassembly, and impair mitosis, thereby blocking progression through M phase of the cell cycle and facilitating apoptosis.

In spite of the undoubted overall clinical success of the taxoids, some tumors display resistance to these drugs. This drug resistance may be an innate feature of a tumor, or may develop in the tumor over time. Three main mechanisms of drug-resistance have been reported: (i) point mutations of the tubulin gene, (ii) selection of tubulin isoforms with low binding to taxanes, and (iii) expression of the multidrug-resistance (MDR) phenotype mediated by the P-glycoprotein (P-gp) efflux pump encoded by the *mdr1* gene. Mechanism (iii) may explain the innate resistance to Taxol® and Taxotere® in tumors that often inherently express P-gp, such as colon and kidney cancers.

The combinations or associations according to the invention enable the phenomena of pleiotropic resistance or "multi-drug resistance" to be avoided or delayed.

The preparation of Taxol®, Taxotere® and their derivatives form the subject, for example, of European Patents EP 0,253,738 and EP 0,253,739 and International Application PCT WO 92/09,589.

It has now been found, and this forms the subject of the present invention, that the efficacy of Taxotere® may be considerably improved when they are administered in combination with at least one substance which is therapeutically useful in anticancer treatments and has a mechanism identical to or different from these taxanes.

More especially, the invention relates to combinations of Taxotere® with the cyclin-dependent kinase, flavopiridol.

Cyclin-dependent kinases (CDKs) are important regulators that control the timing and coordination of the cell cycle. CDKs form reversible complexes with their obligate cyclin partners to control transition through key junctures in the cell cycle. For example, the activated CDK4-cyclin D1 complex controls progression through the G1 phase while the CDK1-cyclin B1 complex controls entry into the mitotic phase of the cell cycle. Endogenous cyclin dependent kinase inhibitory proteins (CDKIs) are known which bind either the CDK or cyclin component and inhibit the kinase activity. In many tumors such as melanomas, pancreatic and esophageal cancers, these natural CDKIs are either absent or mutated. Thus, selective CDK inihibitors may prove to be effective chemotherapeutic agents.

Flavopiridol (*cis*-5,7-dihydroxy-2-(2-chlorophenyl)-8-[4-(3-hydroxy-1-methyl)-piperidinyl]-1-benzopyran-4-one) is a synthetic flavone that has been shown to have antitumor activity against various tumor cells lines such as human lung carcinoma, breast carcinoma, and also inhibits tumor growth in xenograft models. It has been shown to induce arrest in both the G1 and G2 phases of the cell cycle. Flavopiridol is a potent and selective inhibitor of the CDKs, and its antitumor activity is related to its CDK inhibitory activity. Studies have shown that its tumor cell growth inhibitory activity occurs in a cell cycle specific manner. *See* Bioorg. & Med. Chem. Letters 10:1037-1041(2000).

Taxotere® and flavopiridol have differing mechanisms which can improve the efficacy of each. The improved efficacy of a combination according to the invention may be demonstrated by determination of the therapeutic synergy. A combination manifests therapeutic synergy if it is therapeutically superior to one or other of the constituents used at its optimum dose (T.H. Corbett et al., Cancer Treatment Reports, 66: 1187 (1982)).

To demonstrate the efficacy of a combination, it may be necessary to compare the maximum tolerated dose of the combination with the maximum tolerated dose of each of the separate constituents in the study in question. This efficacy may be quantified, for example, by the $\log_{10}$ cell kill, which is determined according to the following formula:

$$\log_{10} \text{ cell kill} = \text{T-C (days)}/3.32 \times T_d$$

in which T - C represents the time taken for the cells to grow, which is the mean time in days for the tumors of the treated group (T) and the tumors of the treated group (C) to have reached a predetermined value (1 g for example), and $T_d$ represents the time in days needed for the volume of the tumor to double in the control animals (T.H. Corbett et al., Cancer, 40, 2660-2680 (1977); F.M. Schabel et al., Cancer Drug Development, Part B, Methods in Cancer Research, 17, 3-51, New York, Academic Press Inc. (1979)). A product is considered to be active if $\log_{10}$ cell kill is greater than or equal to 0.7. A product is considered to be very active if $\log_{10}$ cell kill is greater than 2.8.

The combination, used at its own maximum tolerated dose, in which each of the constituents will be present at a dose generally not exceeding its maximum tolerated dose, will manifest therapeutic synergy when the $\log_{10}$ cell kill is greater than the value of the $\log_{10}$ cell kill of the best constituent when it is administered alone.

The efficacy of the combinations on solid tumors may be determined experimentally in the following manner:

The animals subjected to the experiment, generally mice, are subcutaneously grafted bilaterally with 30 to 60 mg of a tumor fragment on day zero. The animals bearing tumors are mixed before being subjected to the various treatments and controls. In the case of treatment of advanced tumors, tumors are allowed to develop to the desired size, animals having insufficiently developed tumors being eliminated. The selected animals are distributed at random to undergo the treatments and controls. Animals not bearing tumors may also be subjected to the same treatments as the tumor-bearing animals in order to be able to dissociate the toxic effect from the specific effect on the tumor. Chemotherapy generally begins from 3 to 22 days after grafting, depending on the type of tumor, and the animals are observed every day. The different animal groups are weighed 3 or 4 times a week until the maximum weight loss is attained, and the groups are then weighed at least once a week until the end of the trial.

The tumors are measured 2 or 3 times a week until the tumor reaches approximately 2 g, or until the animal dies if this occurs before the tumor reaches 2 g. The animals are autopsied when sacrificed.

The antitumor activity is determined in accordance with the different parameters recorded in Tables I and II.

For a study of the combinations on leukemias, the animals are grafted with a particular number of cells, and the antitumor activity is determined by the increase in the survival time of the treated mice relative to the controls. The product is considered to be active if the increase in survival time is greater than 27%, and is considered to be very active if it is greater than 75% in the case of P388 leukemias.

In the Examples which follow, mice were grafted with mammary adenocarcinoma MA 13/C and treated with combinations of Taxotere® and flavopiridol having different schedules of administration and different modes of administration. Both Taxol and Taxotere, as well as flavopiridol may be administered orally as well as intravenously. Some of these schedules show clear therapeutic synergy.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only.

Example 1

In order to determine the activity of each constituent of the combination, Taxotere® and flavopiridol were given alone to MA 13/C bearing mice. Taxotere® was administered intravenously on days 15 and 21; the dose on each day was 30 mg/kg for a total dosage of 60 mg/kg. Used alone, this dosage resulted in a $\log_{10}$ cell kill of 4.7 and a complete response in all 5 of the mice so treated. Administration of flavopiridol alone on days 15 and 21 in a dose of 6 mg/kg each day, or a total dosage of 12 mg/kg, resulted in no compete responses in the 5 mice tested.

The compounds were then combined in two ways and tested in an intermittent schedule. In the first combination, flavopiridol was given first on days 15 and 20 (total dosage 9 mg/kg) and Taxotere® was given on the following days 16 and 21 (total dosage 45 mg/kg). When given in this fashion, the $\log_{10}$ cell kill was 5.5 and resulted in 5 of 5 complete responses in the mice treated. In this intermittent schedule, the $\log_{10}$ cell kill was better than Taxotere® given alone and still resulted in 5 of 5 complete responses despite the fact that 25 % less Taxotere® and 25 % less flavopiridol were administered in this combination than in the control.

When the constituents of the combination were reversed and Taxotere® was given first on days 15 and 20 and flavopiridol was given on days 16 and 21 in the same amounts, the $\log_{10}$ cell kill was slightly less than Taxotere® alone, but again there were 5 of 5 complete responses. Table 1 illustrates these results and shows that the combination of flavopiridol and Taxotere® administered in 25 % lesser amounts had an efficacy similar to Taxotere® alone.

Table I

| Flavopiridol- Taxotere® Combination in MA13/C bearing mice : 24 hours apart | | | | | | |
|---|---|---|---|---|---|---|
| IV Agent | Schedule | HNTD *(DT)* mg/kg | | % bwl | lck | Comments |
| | | Docetaxel | Flavo | (nadir) | | |
| Taxotere® | 15,21 | 30.0 *(60.0)* | - | 8 (27) | 4.7 | 5/5 CR HDE |

(continued)

| Flavopiridol- Taxotere® Combination in MA13/C bearing mice : 24 hours apart | | | | | | |
|---|---|---|---|---|---|---|
| IV Agent | Schedule | HNTD (DT) mg/kg | | % bwl | lck | Comments |
| | | Docetaxel | Flavo | (nadir) | | |
| Flavopiridol | 15,21 | - | 6.0 (12.0) | 6 (16) | 0.4 | 0/5 CR |
| Combination 24 h apart Flavo 1st Taxotere | 15,20 16,21 | 22.5 (45.0) | 4.5 (9.0) | 7 (22) | 5.5 | 5/5 CR HDE |
| Taxotere 1st | | 22.5 (45.0) | 4.5 (9.0) | 7 (25) | 4.4 | 5/5 CR HDE |
| Td = 2.2 days ; time for control to reach 1 g = 20.3 days ; median burden at start of therapy = 130-160 mg CR = complete response; IV = intravenous; bwl = body weight loss at nadir ; (DT) = total dose; HNTD = highest non-toxic dose ; lck = $\log_{10}$ cell kill | | | | | | |

## Example 2

The days on which flavopiridol was administered were increased, and it was discovered that when the MA 13/C bearing mice were exposed to similar doses of flavopiridol given over 8 days in a 10 day cycle while Taxotere® was given on the first and last day of the ten day cycle, there was clear synergy.

Table 2 below gives the highest non-toxic total dose of each component alone - 96.8 mg/kg of Taxotere® and 23.2 mg/kg of flavopiridol. When the constituents were administered in combination, with Taxotere® being administered on days 14 and 23 and flavopiridol on days 14-17 and 20-23, three combinations were clearly synergistic and a fourth was equal in efficacy to Taxotere® alone. All four combinations resulted in 6 of 6 complete responses; i.e., 100 % complete responses.

A 3-arm dose-response study was performed in C3H/HeN mice bearing measurable tumors at start of therapy (230 mg). The model chosen was a murine mammary adenocarcinoma MA13/C, selected on the basis of its chemosensitivity to docetaxel. Mice were treated with Flavo (i.e. once a day, day 14 to 17, and day 20-23 post tumor implantation), or docetaxel (i.e. on days 14 and 23), or their combination. Results: At the highest non-toxic dose (HNTD, 2.9 mg/kg/ dose, total dose of 23.2 mg/kg), Flavo administered IV as a single agent was inactive with a - 0.4 log cell kill net (log cell kill net = tumor growth delay - treatment duration / 3.32 x tumor doubling time), and no complete regression (CR). The HNTD of docetaxel alone (48.1 mg/kg/injection, total dose of 96.8 mg/kg) was found very active (3 log cell kill net, 6/6 CR). Clear synergy was obtained at the highest non-toxic combination (Flavo at 1.93 mg/kg/dose and docetaxel at 53.2 mg/kg/injection) with a 7.6 log cell kill net and 6/6 CR. This combination was well tolerated inducing a 13 % body weight loss at nadir occurring 6 days post last treatment. Synergy was retained on 2 additional lower dose levels compared to docetaxel HNTD. This synergy was also observed when Flavo was administered orally.

## TABLE II

| Flavopiridol - Taxotere® Combination Mammary adenocarcinoma MA13/C "repeated exposure" | | | | | |
|---|---|---|---|---|---|
| IV agents mg/kg/dose (total dose) | | % bwl | lck Gross | CR | Comments |
| Taxotere d14,23 | Flavopiridol d14-17,20-23 | (nadir) | | | |
| 78.1 (156.2) 48.1 (96.8) | - - | 20 (29) 8 (28) | - 4.4 | - 6/6 | 2/6 DD HNTD |
| - - | 4.8 (38.4) 2.9 (23.2) | >20 3 (18) | - 1.0 | - 0/6 | 4/5 DD HNTD |

(continued)

| Flavopiridol - Taxotere® Combination Mammary adenocarcinoma MA13/C "repeated exposure" | | | | | |
|---|---|---|---|---|---|
| IV agents mg/kg/dose (total dose) | | % bwl | lck | CR | Comments |
| Taxotere d14,23 | Flavopiridol d14-17,20-23 | (nadir) | Gross | | |
| - | 1.75 *(14)* | +10 (24) | 0.0 | 0/6 | |
| 48.4 *(96.8)* | 2.9 *(23.2)* | 15 (27) | - | - | 2/5 DD |
| 53.2 *(106.4)* | 1.93 *(15.44)* | 13 (29) | 9.0 | 6/6 | HNTD |
| 43.6 *(87.2)* | 1.6 *(12.8)* | 9 (29) | 7.0 | 6/6 | |
| 36.3 *(72.6)* | 1.31 *(10.48)* | 3 (29) | 5.1 | 6/6 | |
| 31.5 *(63.0)* | 1.14 *(9.12)* | 6 (28) | 4.4 | 6/6 | |
| 24.2 *(48.4)* | 0.88 *(7.04)* | 5 (19) | 2.0 | 2/6 | |
| Td = 2.2 days. Time for control to reach 1 g = 17.8 days ; median tumor burden at start of therapy 210-260 mg ; CR = complete response; IV = intravenous; bwl = body weight loss at nadir ; (DT) = total dose ; HNTD = highest non-toxic dose ; lck = $\log^{10}$ cell kill. | | | | | |

Example 3

Taxotere was injected IV on days 14 and 25 post tumor implantation. Flavo was orally administered once a day from day 14 to 18 and from day 21 to 25.

FLAVOPIRIDOL (po) - DOCETAXEL (iv)

| Prolonged exposure MA13/C | | | | | |
|---|---|---|---|---|---|
| Agent | Schedule | HNTD (DT) mg/kg TXT      Flavo | % bwl (nadir) | lck | Comments |
| TXT Flavo | 14,25 14-18, 21-25 | 30 (60)       - 2.7 (27) | 3.6 (33) 11.6 (26) | 0.9 0.1 | HNTD HNTD |
| Combination TXT Combination Flavo | 14,25 14-18, 21-25 | 45 (90)       4.5 (45) | 9.7 (19) | □5 | HNTC |
| ≅320 mg - tumor weight on day 14 (BCM-1252) - experiment no. | | | | | |

As in Example 2, the combination of docetaxel and repeated daily Flavo was found more active than either of the single agents, at equitoxic dosages. Synergy is shown by the log 4 increase in cells killed.
Other embodiments of the invention will be apparent to those skilled in the art from consideration of the specification and practice of the invention disclosed herein. It is intended that the specification and examples be considered as exemplary only

**Claims**

1. A pharmaceutical composition having therapeutic synergy in the treatment of neoplastic disease comprising Taxotere® and flavopiridol.

2. The pharmaceutical composition of claim 1, wherein the constituents of the composition are administered separately and spaced out over time.

3. The pharmaceutical composition of claim 1, wherein the neoplastic disease is breast cancer.

4. The pharmaceutical composition of claim 1, wherein the neoplastic disease is lung cancer.

5. The pharmaceutical composition of claim 1 wherein the flavopiridol is administered orally.


**Patentansprüche**

1. Pharmazeutische Zusammensetzung mit therapeutischem Synergismus bei der Behandlung von neoplastischen Erkrankungen, enthaltend Taxotere® und Flavopiridol.

2. Pharmazeutische Zusammensetzung nach Anspruch 1, wobei die Bestandteile der Zusammensetzung getrennt und zeitlich versetzt verabreicht werden.

3. Pharmazeutische Zusammensetzung nach Anspruch 1, wobei es sich bei der neoplastischen Erkrankung um Brustkrebs handelt.

4. Pharmazeutische Zusammensetzung nach Anspruch 1, wobei es sich bei der neoplastischen Erkrankung um Lungenkrebs handelt.

5. Pharmazeutische Zusammensetzung nach Anspruch 1, wobei das Flavopiridol oral verabreicht wird.


**Revendications**

1. Composition pharmaceutique présentant une synergie thérapeutique dans le traitement d'une maladie néoplasique, comprenant du Taxotere® et du flavopiridol.

2. Composition pharmaceutique selon la revendication 1, où les constituants de la composition sont administrés séparément et de manière espacée dans le temps.

3. Composition pharmaceutique selon la revendication 1, où la maladie néoplasique est le cancer du sein.

4. Composition pharmaceutique selon la revendication 1, où la maladie néoplasique est le cancer du poumon.

5. Composition pharmaceutique selon la revendication 1, où le flavopiridol est administré par voie orale.

## REFERENCES CITED IN THE DESCRIPTION

**Patent documents cited in the description**

- EP 0253738 A
- EP 0253739 A
- WO 9209589 A

**Non-patent literature cited in the description**

- *Bioorg. & Med. Chem. Letters,* 2000, vol. 10, 1037-1041
- **T.H. CORBETT et al.** *Cancer Treatment Reports,* 1982, vol. 66, 1187
- **T.H. CORBETT et al.** *Cancer,* 1977, vol. 40, 2660-2680
- **F.M. SCHABEL et al.** Cancer Drug Development, Part B, Methods in Cancer Research. Academic Press Inc, 1979, vol. 17, 3-51